# EUROPEAN PATENT SPECIFICATION

(11) **EP 4 135 681 B1**
(45) Date of publication and mention of the grant of the patent: **25.06.2025**
(21) Application number: 21723640.5
(22) Date of filing: 16.04.2021
(51) Int. Cl.: A61K 31/215, A61P 31/14, A61P 31/12, A61P 31/16

(54) **ANTIVIRAL USE OF PLEUROMUTILINS**
ANTIVIRALE VERWENDUNG VON PLEUROMUTILINEN
UTILISATION ANTIVIRALE DE PLEUROMUTILINES

(30) Priority: 17.04.2020 US 202063011474 P
(43) Date of publication of application: 22.02.2023
(73) Proprietor: Nabriva Therapeutics GMBH, 1110 Wien (AT)
(72) Inventor: PAUKNER, Susanne, 1160 Vienna (AT); WICHA, Wolfgang, 2460 Bruck an der Leitha (AT); GELONE, Steven Peter, Flourtown, PA 19031 (US); ASCHER, Gerd, 1190 Vienna (AT); RIEDL, Rosemarie, 1220 Vienna (AT)
(74) Representative: Schwarz & Partner Patentanwälte GmbH
(86) International application number: PCT/EP2021/025139
(87) International publication number: WO 2021/209173

(56) References cited:
- WO-A1-2008/113089
- WO-A1-2009/106839
- ALEXANDER ELIZABETH ET AL: "Oral Lefamulin vs Moxifloxacin for Early Clinical Response Among Adults With Community-Acquired Bacterial Pneumonia: The LEAP 2 Randomized Clinical Trial", JAMA (JOURNAL OF THE AMERICAN MEDICAL ASSOCIATION), vol. 322, no. 17, 5 November 2019 (2019-11-05), pages 1661 - 1671, XP009526014
- ZHIBAO CHEN ET AL: "Preventive Effects of Valnemulin on Lipopolysaccharide-Induced Acute Lung Injury in Mice", INFLAMMATION, KLUWER ACADEMIC PUBLISHERS-PLENUM PUBLISHERS, NE, vol. 33, no. 5, 11 March 2010 (2010-03-11), pages 306 - 314, XP019819650, ISSN: 1573-2576

## Description

The present invention relates to a novel therapeutic use of Pleuromutilins.

Pleuromutilin, a compound of formula 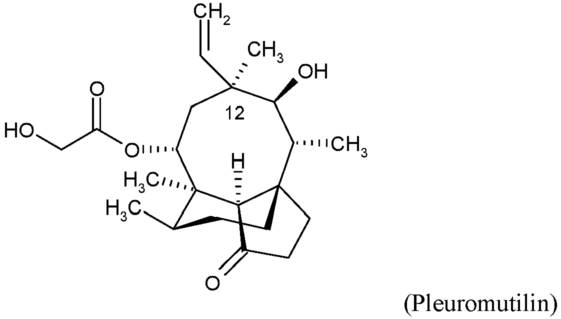 is a naturally occurring antibiotic, produced e.g. by the basidiomycetes *Pleurotus mutilus* and *P. passeckerianus,* see e.g. The Merck Index, 12th edition, item 7694.

A number of further Pleuromutilins having the principle ring structure of Pleuromutilin and being substituted at the primary hydroxy group have been developed, e.g. as antibacterials. Due to their pronounced antibacterial activity, a group of Pleuromutilin derivatives, amino-hydroxy-substituted cyclohexylsulfanylacetylmutilins, as disclosed in WO 2008/113089, have been found to be of particular interest. As described in WO 2008/113089 14-O-{[(4-Amino-2-hydroxy-cyclohexyl)-sulfanyl]-acetyl}-mutilins are particularly useful compounds because of their activity against Gram-positive and Gram-negative bacteria.

Pharmaceutical active compounds derived from Pleuromutilin (semi synthetic compounds) are inhibitors of ribosomal protein synthesis in bacteria. Representatives of semisynthetic Pleuromutilins for human use are Retapamulin (approved as AltargoP^{®}, AltabaxP^{®}), a topical agent approved for short term treatment of impetigo and infected small lacerations, abrasions or sutured wounds, and Lefamulin (approved as Xenleta^{®}) for the treatment of adults with community-acquired bacterial pneumonia (CABP). Tiamulin (Denagard^{®}) and Valnemulin (Econor^{®}) are two other semi-synthetic Pleuromutilin derivatives which have been used systemically as antibiotics in veterinary medicine for many years.

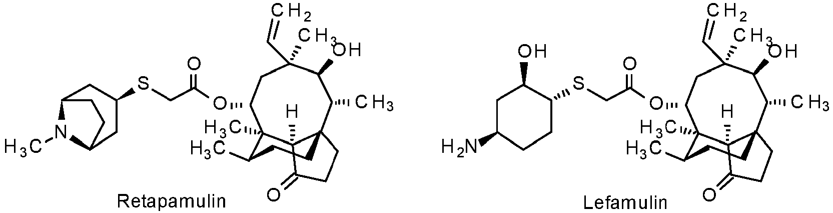

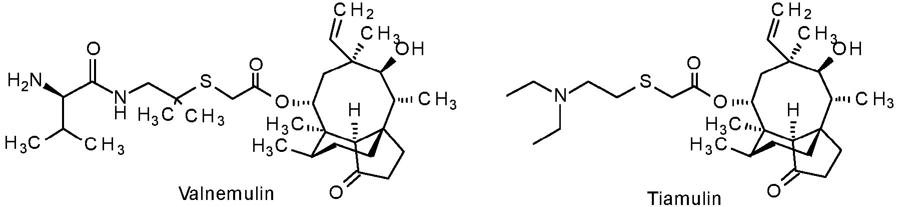

Approved semisynthetic compounds derived from Pleuromutilin have shown excellent activity against bacterial organisms which include inter alia *Streptococcus pneumoniae, Haemophilus influenzae, Staphylococcus aureus* (including MRSA), *Moraxella catarrhalis, Legionella pneumophila, Chlamydophila pneumoniae* and *Mycoplasma pneumoniae.*

Viral diseases are one of the leading causes of morbidity and mortality in the world. Respiratory viruses such as influenza, respiratory syncytial virus, certain adenoviruses, rhinoviruses and corona viruses and in particular the newly emerged severe acute respiratory syndrome coronavirus 2 (SARS-CoV-2; COVID-19) have a significant impact on public health.

In Asheshov, Igor N. et. al., Antibiotics & Chemotherapy 4/4 (1954), 380-394, for the first time the antiviral activity of Pleuromutilins was described with antiviral activity of Pleuromutilin itself for an influenza A virus strain (PR8) at a concentration of 2 mg/mL. In contrast, Pleuromutilin did not show antiviral activity for polio virus in this study.

Furthermore, in Alacórn, Balbino et.al., Antiviral Research, 4 (1984), 231-243, the antiviral activity of Pleuromutilin against both, DNA and RNA viruses, in particular herpes simplex type 1 (HSV-1) virus at a test compound concentration that conferred a 50% protection of the cytopathic effect induced by HSV-1 (CPE50) of 40 µM (15 µg/mL) and activity against vesicular stomatitis virus (VSV) is described.

In WO 2009/106839 the use of Tiamulin as an antiviral agent is claimed, with effect of Tiamulin on influenza A virus, porcine reproductive and respiratory syndrome virus (PRRSV) type 1 and 2 in a viral up-take assay 4 hours post inoculation with the virus at Tiamulin concentrations of 0.1-10 µg/mL compared to Valnemulin and the effect of Tiamulin on endosomal pH exemplified. Valnemulin did not exhibit antiviral activity and it was stated that other Pleuromutilin antibiotics have not been found to have an effect on viruses.

Alteration of the endosomal or lysosomal pH by Tiamulin and associated prevention of fusion of the viral membrane with endo- and lysosomes, which is a pre-requisite for viral entry, was described as potential mode-of-action.

CN 103204787B and CN 103242210 both disclose further Pleuromutilin derivatives and generally mention their use in antiviral drugs, without, however, disclosing any actual proof for an antiviral action.

Certain statements about potential antiviral and anti-inflammatory effects of Lefamulin were made in the "Q1 2020 Nabriva Therapeutics PLC Earnings Call" of May 11, 2020, (a transcript of which is available under https://www.yahoo.com/news/edited-transcript-nbrv-oq-earnings-144108621.html, downloaded June 10, 2020 as well as in a press release of May 11, 2020 (https://investors.nabriva.com/news-releases/news-release-details/nabriva-therapeutics-reports-first-quarter-2020-financial), downloaded May 28, 2020.

### SUMMARY OF THE INVENTION

Surprisingly, it was now found that the Pleuromutilin derivatives disclosed in WO 2008/113089 A1 are effective against viruses and, thus, effective against diseases mediated by viruses.

Therefore, in a first aspect the present invention relates to a compound as defined in claims 1 to 6, in particular Lefamulin, or a pharmaceutically acceptable salt, or solvate thereof, for the specific use in the treatment or prevention of a disease mediated by a virus.

In a further aspect, the present invention relates to a method of treatment or prevention of a disease mediated by a virus, comprising administering a compound as defined in any of claims 1 to 6, in particular Lefamulin, or a pharmaceutically acceptable salt, or solvate thereof to a subject in need of such treatment.

### BRIEF DESCRIPTION OF THE DRAWINGS

Figure 1 demonstrates the effect of Lefamulin against alpha corona virus 229E (HCoV-229E) in MRC-5 cells 6 days post infections with the virus.
Figure 2 demonstrates the effect of Tiamulin in the same assay.
Figure 3 demonstrates the effect of Remdesivir in the same assay.
Figure 4 demonstrates the effect of Lefamulin against respiratory syncytial virus type A in HEp2 cells 6 days post infections with the virus.

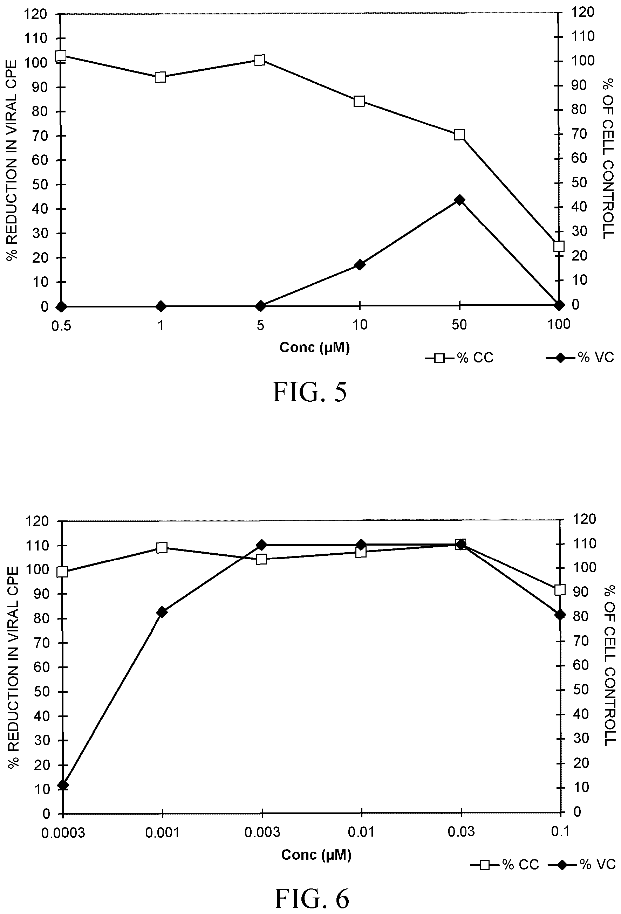

Figure 5 demonstrates the effect of Tiamulin in the same assay.
Figure 6 demonstrates the effect of TMC353121 in the same assay.
Figures 7A to 7D demonstrate the effect of Lefamulin and Oseltamivir on clinical signs in an Influenza infection mouse model, in particular on the body weight (A), clinical score (B), percentage survival (C), and lung sample histopathology scoring (D).
Figure 8 demonstrates the effect of Lefamulin and Oseltamivir on lung viral titer in the Influenza infection mouse model determined as 50% tissue culture infective dose (TCID₅₀) in MDCK cells.

### DETAILED DESCRIPTION OF THE INVENTION

Lefamulin is the INN for a compound of generic formula (I), more particular, Lefamulin is a compound of formula (VII) 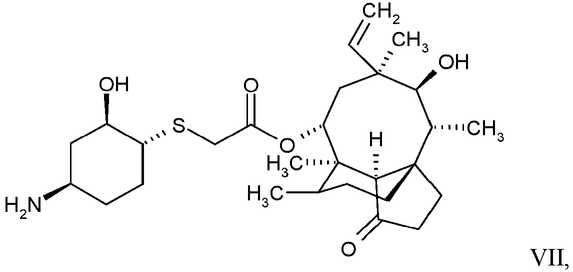 i.e. 14-O-{[(1*R,* 2*R*, 4*R*)-4-amino-2-hydroxy-cyclohexylsulfanyl]-acetyl}-mutilin (also known as "BC-3781").

In the following, the term "Lefamulin", when generally used without additional explanation, is intended to encompass both Lefamulin in free base form, as well as its salts and solvates.

Lefamulin has been developed for systemic use to treat serious bacterial infections in humans and was approved for medical use in the United States in 2019 to treat adults with community-acquired bacterial pneumonia (CABP).

The present invention refers to the treatment and prevention of a disease mediated by viruses, e.g. a viral disease or viral infection.

The results of the experiments show that besides its antibacterial activity, Lefamulin is also actively reducing the cytopathic effect mediated by different viruses. This antiviral effect was particularly shown for such viruses that are characterized in that they are positive- or negative sense single-stranded RNA viruses. Antiviral activity was shown for both enveloped and non-enveloped viruses, in particular several enveloped positive- or negative sense single-stranded RNA viruses (such as Coronaviridae, Paramyxoviridae, Orthomyxoviridae, and Flaviviridae). Moreover, some of the investigated viruses, including measles virus are known for a transmission involving the respiratory route, in particular airborne transmission. Corona virus and Respiratory Syncytial Virus also cause infections of the respiratory tract in humans.

In a preferred embodiment of the present invention, the virus is a positive- or negative-sense single-stranded RNA virus,
preferably the virus is selected from the group consisting of
- Coronaviridae including in particular human coronavirus,
- Paramyxoviridae including in particular Paramyxovirinae, such as Measles virus, and Pneumovirinae, such as Respiratory Syncytial Virus,
- Orthomyxoviridae including in particular Influenza virus,
- Flaviviridae including in particular Dengue virus and Zika virus, and
- Picornaviridae including in particular Rhinovirus.

In an other embodiment, the disease is an airborne disease. An airborne disease is mediated by a virus transmitted by the air.

Viral infections can affect various organs. In a preferred embodiment of the present invention, the disease is a respiratory disease, including upper and lower respiratory infections, in particular lower respiratory infections.

In particular, the disease is an acute respiratory syndrome, such as Influenza, Severe Acute Respiratory Syndrome (SARS), Middle East Respiratory Syndrome (MERS) or COVID-19.

In a further embodiment of the present invention the disease is mediated by a virus selected from the group consisting of viruses of the virus families Coronaviridae, in particular a corona virus such as SARS-CoV, SARS-CoV2, MERS-CoV or HCoV-229E, Orthomyxoviridae, in particular an Influenza virus such as Influenza A and B viruses, Paramyxoviridae in particular Respiratory Syncytial Virus and Adenoviridae, in particular Adenovirus.

In an embodiment, the virus is a corona virus, in particular selected from the group consisting of SARS-CoV, SARS-CoV2, MERS-CoV, and HCoV-229E as well as mutations thereof. Such corona viruses are known to cause (severe) acute respiratory syndromes, such as SARS, MERS or COVID-19.

Treating, treatment or to treat as understood herein includes on one hand the complete curing, curation or to cure a condition (the disease mediated by a virus) such that it comes to its end and on the other hand also ameliorating, amelioration or to ameliorate a condition such that its symptoms are reduced at least partially or individually.

Treatment typically includes administering a compound as used according to the present invention to a subject in need thereof, i.e. a subject being diagnosed to have a disease mediated by a virus.

Preventing, prevention, or to prevent includes administering a compound before a condition is diagnosed or before onset of (all) disease symptoms of the condition.

Prevention of diseases mediated by viruses includes administering the compounds before onset of disease symptoms. Prevention may be considered after a subject has been infected with a virus but has not shown any symptoms, or wherein a subject has been exposed and/or is prone to exposition to a virus.

The appropriate dosage of the compound to be administered according to the present invention, in particular Lefamulin, will, of course, vary depending upon, for example, the individual host, the mode of administration and the nature and severity of the conditions being treated. However, in general, for satisfactory results in larger mammals, for example humans, an indicated daily dosage is in the range from about 0.5 mg to 3 g of a compound used according to the present invention conveniently administered, for example, in divided doses up to four times a day.

The compound used according to the present invention may be administered by any conventional route, for example enterally, e.g. including nasal, buccal, rectal, oral administration; parenterally, e.g. including intravenous, intramuscular, subcutaneous administration; or topically, e.g. including pulmonary, epicutaneous, intranasal, intratracheal administration, e.g. in form of coated or uncoated tablets, capsules, injectable solutions or suspensions, e.g. in the form of ampoules, vials, in the form of ointments, creams, gels, pastes, inhaler powder, foams, tinctures, lip sticks, drops, sprays, or in the form of suppositories, e.g. in analogous manner to the antibiotic agent tobramycin or macrolides, such as erythromycins, e.g. clarithromycin or azithromycin.

Preferably, the compound used according to the present invention is administered via inhalation, via intravenous or subcutaneous injection, or orally.

Preferred pharmaceutical compositions of Lefamulin for injection are disclosed in WO 2016/202788 A1.

The compound used according to the present invention, in particular Lefamulin, may be administered in the form of a pharmaceutically acceptable salt, e.g. an acid addition salt, or in free form, optionally in the form of a solvate.

In one embodiment, the compound is in the form of a salt and/or a solvate.

A salt of a compound used according to the present invention includes an acid addition salt.

Pharmaceutically acceptable acid addition salts include salts of a compound used according to the present invention with an acid, e.g. hydrogen fumaric acid, fumaric acid, tartaric acid, ethane-1,2-disulphonic acid, maleic acid, naphthalin-1,5-sulphonic acid, acetic acid, malic acid, lactic acid, i.e. L-lactic acid, succinic acid, salicylic acid, azelaic acid, 2-[(2,6-dichlorophenyl)amino]benzene acetic acid, hydrochloric acid, deuterochloric acid, preferably hydrochloric acid, acetic acid, L-lactic acid, and maleic acid.

Of these, in the case of Lefamulin, the acetate salt of Lefamulin is especially preferred.

Preferred crystalline forms of Lefamulin as well as crystalline salt forms of Lefamulin are disclosed in WO 2011/146954 A1. Of these, the acetate salt of Lefamulin in crystalline Form B as disclosed in WO 2011/146954 A1 is especially preferred.

The present invention also provides the Lefamulin in its form as acid addition salt with itaconic acid, in particular Lefamulin itaconate. Lefamulin itaconate is disclosed herein as a new compound (Example 12). Itaconic acid can be deprotonated to the anions hydrogen itaconate and itaconate. The acid addition salt comprising Lefamulin as cation and an anion derived from itaconic acid is expected to be useful as antiviral agent.

The compound used according to the present invention, in particular Lefamulin, may be used for the pharmaceutical treatment contemplated herein alone or in combination with one or more other pharmaceutically active agents. Such other pharmaceutically active agents include e.g. other antiviral agents. Such other antiviral agents may preferably be selected from the group consisting of nucleoside and nucleotide analogues and RNA polymerase inhibitors, e.g. Remdesivir or Ribavirin, viral protease inhibitors such as Lopinavir or Ritonavir, viral neuraminidase inhibitors, such as Oseltamivir, and other agents used in antiviral therapy such as Hydroxychloroquine, interferons (interferon alfa and/or beta), or other broad-spectrum antiviral agents.

Combinations include fixed combinations, in which two or more pharmaceutically active agents are in the same formulation; kits, in which two or more pharmaceutically active agents in separate formulations are sold in the same package, e.g. with instruction for coadministration; and free combinations in which the pharmaceutically active agents are packaged separately, but instruction for simultaneous or sequential administration are given.

A pharmaceutical composition comprising a compound used according to the present invention, in particular Lefamulin may in addition comprise at least one pharmaceutically acceptable excipient, e.g. carrier or diluent, e.g. including fillers, binders, disintegrators, flow conditioners, lubricants, sugars and sweeteners, fragrances, preservatives, stabilizers, wetting agents and/or emulsifiers, solubilizers, salts for regulating osmotic pressure and/or buffers.

Such pharmaceutical compositions may be manufactured according, e.g. analogously, to a method as conventional, e.g. by mixing, granulating, coating, dissolving, spray drying, or lyophilizing processes. Unit dosage form may contain, for example, from about 0.5 mg to about 3000 mg, such as 10 mg to about 600 mg.

A subject in need of a treatment as contemplated by the present invention may be any living subject suffering from a disease mediated by a virus. Especially, the subject may be a human or an animal.

### Examples

Herein, including the examples, the following abbreviations are used:
- ¹H-NMR: proton nuclear magnetic resonance spectroscopy
- °C: degrees Celsius
- µM: micromolar concentration
- BALB/c: laboratory-bred mouse strain
- BC-3781: lefamulin
- CC: Cell Control
- CoV: corona virus
- CPE: cytopathic effects, in particular virus-induced
- DMEM: Dulbecco's modified Eagle's medium
- DMF: N,N-dimethylformamide
- DMSO: dimethylsulfoxide
- EC₅₀: Half maximal (fifty-percent) effective concentration
- eq: equivalents
- FBS: Fetal bovine serum
- HeLa: immortal human epithelial cell line
- HEp2: human epithelial cell line
- Huh7: human liver cell line
- MDCK: Madin-Darby Canine Kidney cells
- MOI: Multiplicity of infection
- MRC-5: Medical Research Council cell strain 5
- M: molarity
- MS: mass spectrometry
- m/z: mass/charge ratio
- MTBE: methyl-*tert*-butylether
- nm: nanometer
- TC₅₀: Half maximal (fifty-percent) toxic concentration
- TCID₅₀: Fifty-percent (half maximal) tissue culture infective dose
- VC: reduction in viral CPE
- XTT: 2,3-bis-(2-methoxy-4-nitro-5-sulfophenyl)-2H-tetrazolium-5-carboxanilide

### Example 1

**Objective:** The assay measured the inhibition of virus-induced cytopathic effects (CPE) and cell viability following alpha coronavirus 229E (HCoV-229E or CoV_{229E}) in MRC-5 cells 6 days post infections with the virus by various concentrations of Lefamulin (BC-3781).

**Methodology:** MRC-5 cells were seeded in 96-well flat-bottom tissue culture plates (at a density of 3 x 10³ cells per well) and allowed to adhere overnight. Thereafter, diluted test compounds (Lefamulin as acetate, Tiamulin as fumarate) dissolved in DMSO were added to the plate and incubated for 4 hours prior to addition of the virus. The virus was added diluted to a pre-determined titer to yield 85-95% cell killing at 6 days post-infection (MOI of 0.001).

Following incubation at 37°C and at 5% CO₂ for 6 days, cell viability was measured by XTT tetrazolium dye staining. The optical density of the cell culture plate was determined spectrophotometrically at 450 and 650 nm. Percent reduction of the virus-infected cells and the percent cell viability of uninfected drug control wells were calculated to determine the effective concentration at which 50% of cytopathic effect was inhibited (EC₅₀) and the cytotoxic concentration (TC₅₀) using four parameter curve fit analysis. The antiviral compound Remdesivir served as positive control.

### Results:

Surprisingly, Lefamulin reduced the viral CPE by 91.82% at a concentration of 10 µM, which is a concentration that had no cytotoxic effect on the viability of the cell control. The calculated EC₅₀ was 3.87 µM, at which 50% of the viral cytopathic effect was inhibited. At the Lefamulin concentration of 50 µM, Lefamulin displayed a cytotoxic effect; the calculated TC₅₀ was 55.3 µM. The ratio of EC₅₀ and TC₅₀, known also as therapeutic index, was 14.3.

In contrast, Tiamulin at a concentration of 10 µM reduced the viral CPE only by 10.53% and no cytotoxic effect was observed. At the next higher test concentration of 50 µM the CPE was reduced by 81.68% and a cytotoxic effect was observed. The calculated EC₅₀ was 24.4 µM and the calculated TC₅₀ was 62.9 µM. The therapeutic index of Tiamulin was 2.58 and surprisingly much lower than that of Lefamulin.

The antiviral compound Remdesivir was developed as a treatment for Ebola virus, and also is known to have antiviral activity against corona viruses (clinical investigation is ongoing).

Thus, Remdesivir served as positive control herein. Remdesivir showed an EC₅₀ of 0.11 µM, a TC₅₀ of > 5 and a therapeutic index of > 45.5.

| Compound | MRC-5 cells infected with CoV_{229E} | | |
|---|---|---|---|
| | EC₅₀ (µM) | TC₅₀ (µM) | Therapeutic Index |
| Lefamulin | 3.87 | 55.3 | 14.3 |
| Tiamulin | 24.4 | 62.9 | 2.58 |
| Remdesivir | 0.11 | >5.00 | >45.5 |

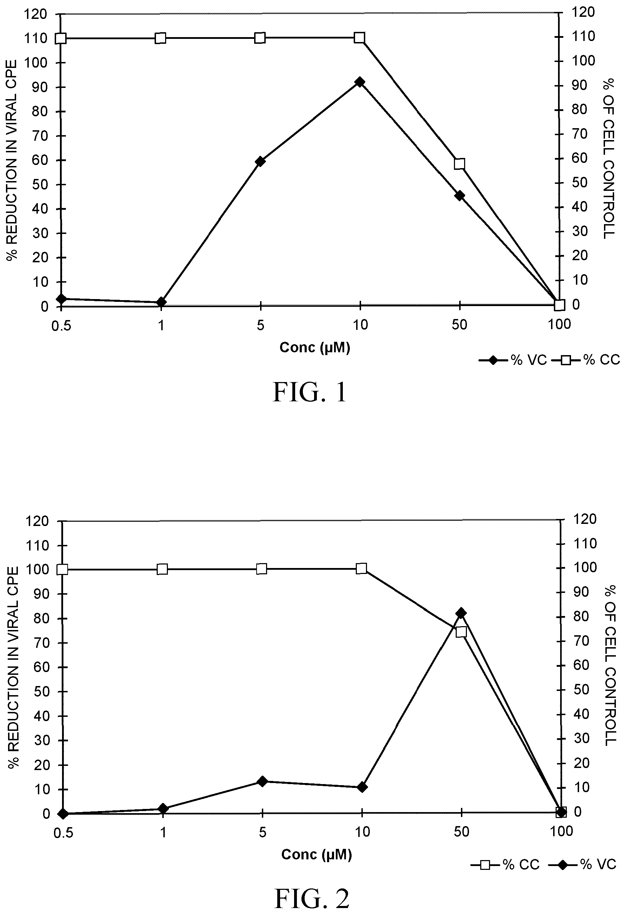

The results are graphically displayed in Figures 1 (Lefamulin), 2 (Tiamulin) and 3 (Remdesivir) (VC....reduction in viral CPE, CC.....Cell Control).

### Example 2:

**Objective:** The assay measured the inhibition of virus-induced cytopathic effects (CPE) and cell viability following alpha coronavirus 229E (HCoV-229E or CoV_{229E}) in MRC-5 cells for Lefamulin at various treatment conditions.

**Method:** The assay was performed in analogy to Example 1 above with the following differences regarding the test candidate. Lefamulin (as acetate) was evaluated using different treatment conditions of 4 h, 1 h or 0 h incubation prior to virus addition and addition 1 h after infection For this particular set of experiments, coronavirus was diluted 1:200 in assay medium and added at 100 µL/well to achieve approximately 90% cell killing in the untreated virus control wells (MOI of 0.001).

### Results:

The antiviral efficacy and cellular toxicity data are summarized in the table below. Lefamulin showed a time-dependent effect on the inhibition of the virus-induced cytopathic effects (CPE). In the treatment setting after viral exposure (1h post-infection) a dose-dependent effect was observed. At a concentration of 50 µM the viral CPE was reduced by 86.83 % (data not shown in detail).

| Treatment condition | MRC-5 cells infected with CoV_{229E} | | |
|---|---|---|---|
| Lefamulin | EC₅₀ (µM) | TC₅₀ (µM) | Therapeutic Index |
| 4 h pretreatment | 2.18 | >50.0 | > 22.9 |
| 1 h pretreatment | 2.16 | >50.0 | > 23,1 |
| 0 h pretreatment | 3.08 | >50.0 | >16.2 |
| 1 h post-infection | 15.1 | >50.0 | >3.31 |

### Example 3

**Objective:** The assay measured the inhibition of virus-induced cytopathic effects (CPE) and cell viability following human respiratory syncytial virus (strain RSV_{A2}) replication in HEp2 cells 6 days post infections with the virus by various concentrations of Lefamulin (BC-3781).

**Methodology:** HEp2 cells were seeded in 96-well flat-bottom tissue culture plates (at a density of 5 x 10³ cells per well) and allowed to adhere overnight. Thereafter, diluted test compounds (Lefamulin as acetate, Tiamulin as fumarate) in DMSO were added to the plate and incubated for 4 hours prior to addition of the virus. The virus was added diluted to a pre-determined titer to yield 85-95% cell killing at 6 days post-infection (MOI of 0.001).

Following incubation at 37°C and at 5% CO₂ for 6 days, cell viability was measured by XTT tetrazolium dye staining. The optical density of the cell culture plate was determined spectrophotometrically at 450 and 650 nm. Percent reduction of the virus-infected cells and the percent cell viability of uninfected drug control wells were calculated to determine the effective concentration at which 50% of cytopathic effect was inhibited (EC₅₀) and the cytotoxic concentration (TC₅₀) using four parameter curve fit analysis. The antiviral compound TMC353121 (RSV fusion inhibitor) served as positive control.

### Results:

Surprisingly, Lefamulin reduced the viral cytopathic effect (CPE) by 92.17% and 100% at concentrations of 10 µM and 50 µM, respectively, which are concentrations that had no cytotoxic effect on the viability of the cell control. The calculated EC₅₀ was 5.34 µM, at which 50% of the viral CPE was inhibited. At the Lefamulin concentration of 100 µM, Lefamulin displayed a cytotoxic effect; the calculated TC₅₀ was 70.7 µM. The ratio of EC₅₀ and TC₅₀, known also as therapeutic index, was 13.2.

In contrast, Tiamulin at a concentration of 10 µM reduced the viral CPE only by 16.76% and a cytotoxic effect (84% viability) was observed at this concentration. At the next higher test concentration of 50 µM the viral CPE was reduced by 43.28% and at the cytotoxic effect was more pronounced (70.0% viability). The calculated EC₅₀ was with >67.9 µM above the calculated TC₅₀ of 67.9 µM. The therapeutic index of Tiamulin therefore could not be calculated. Surprisingly, the antiviral activity and the therapeutic index was much higher for Lefamulin than for Tiamulin.

The antiviral compound TMC353121 was developed as a specific respiratory syncytial virus fusion inhibitor (clinical investigation is ongoing). Thus, TMC353121 served as positive control herein. TMC353121 showed an EC₅₀ of 0.006 µM, a TC₅₀ of > 0.1 µM and a therapeutic index of > 167.

| Compound | HEp2 cells infected with human respiratory syncytial virus (RSVS_{A2}) | | |
|---|---|---|---|
| | EC₅₀ (µM) | TC₅₀ (µM) | Therapeutic Index |
| Lefamulin | 5.34 | 70.7 | 13.2 |
| Tiamulin | >67.9 | 67.9 | -- |
| TMC353121 | 0.0006 | >0.1 | >167 |

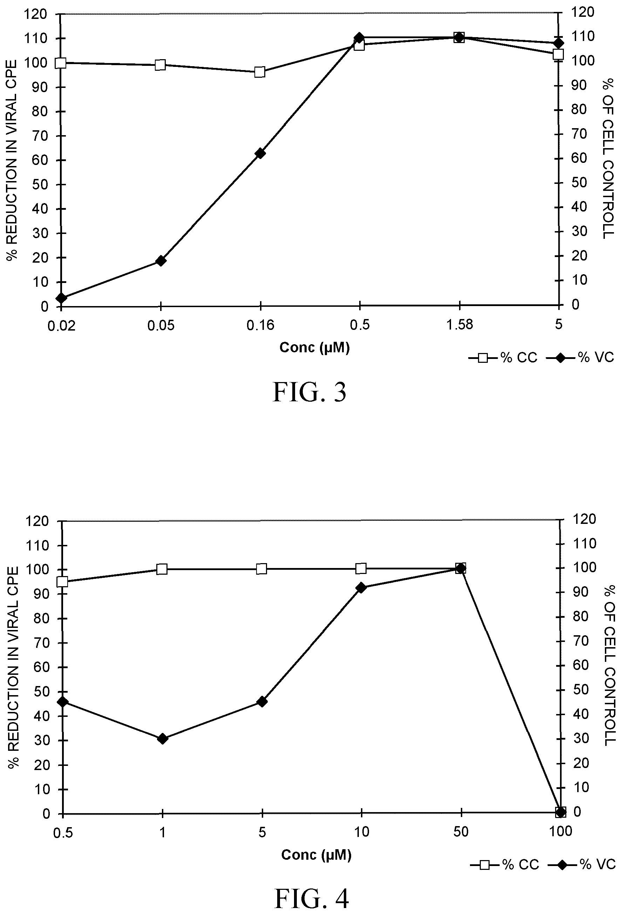

The results are graphically displayed in Figures 4 (Lefamulin), 5 (Tiamulin) and 6 (TMC353121) (VC....Reduction in viral CPE, CC.....Cell Control).

### Example 4

**Objective:** The assay measured the inhibition of virus-induced cytopathic effects (CPE) and cell viability following human respiratory syncytial virus (strain RSV_{A2}) replication in HEp2 cells changing the multiplicity of infection (MOI).

**Methodology:** The assay was performed in analogy to Example 3 above with the following differences regarding the test. The virus was added diluted to a pre-determined titer to yield 85-95% cell killing at 6 days post-infection, and the added amount was adopted to obtain MOIs of 0.003, 0.001, 0.0008, and 0.0004, respectively. Lefamulin (as acetate) was investigated in this study as well as TMC353121 for positive control.

### Results:

The antiviral efficacy and cellular toxicity data are summarized in the tables below. The EC₅₀ value in the low µM range for Lefamulin was reproduced at an MOI of 0.0004. In contrast, a higher MOI, thus higher viral load with respect to the investigated cells, reduced the antiviral effect of Lefamulin. This effect is less pronounced in the highly effective control substance TMC353121.

| MOI / Compound | HEp2 cells infected with human respiratory syncytial virus (RSV_{A2}) | | |
|---|---|---|---|
| Lefamulin | EC₅₀ (µM) | TC₅₀ (µM) | Therapeutic Index |
| 0.003 | > 63.2 | 63.2 | --- |
| 0.001 | 32.3 | 65.9 | 2.04 |
| 0.0008 | 28.7 | 66.60 | 2.32 |
| 0.0004 | 2.34 | 67.4 | 28.8 |

| MOI / Compound | HEp2 cells infected with human respiratory syncytial virus (RSV_{A2}) | | |
|---|---|---|---|
| TMC353121 | EC₅₀ (µM) | TC₅₀ (µM) | Therapeutic Index |
| 0.003 | 0.0002 | >0.1 | 500 |
| 0.001 | 0.0002 | >0.1 | >500 |
| 0.0008 | 0.0003 | >0.1 | >333 |
| 0.0004 | 0.00004 | >0.1 | >2500 |

### Example 5

**Objective:** The assay measured the inhibition of virus-induced cytopathic effects (CPE) and cell viability following replication of the two different respiratory syncytial virus strains RSV A_{LONG} and RSV B₁₈₅₃₇ in HEp2 cells.

**Methodology:** The assay was performed in analogy to Example 3 above with the difference that cells seeded with a density of 5 x 10³ cells per well were incubated with the virus strains RSV A_{LONG} or RSV B₁₈₅₃₇, respectively, following a 4 hour cell pretreatment with the test compound at different concentrations. Virus was diluted and added in an amount yielding MOIs of 0.01 and 0.001 for RSV A_{LONG} and RSV B₁₈₅₃₇, respectively.

### Results:

The antiviral efficacy and cellular toxicity data are summarized in the tables below. The control compound TMC353121 was evaluated in parallel to Lefamulin and yielded an EC₅₀ value of 0.01 nM against the investigated strains of RSV A and RSV B. Lefamulin yielded an EC₅₀ value of 17.7 µM against the RSV B₁₈₅₃₇. Activity against RSV A_{LONG} could not be determined due to the cytotoxicity to HEp2 cells with TC₅₀ values of 71.1 µM in the assay.

| | HEp2 cells infected with respiratory syncytial virus (RSV A_{LONG}) | | |
|---|---|---|---|
| Compound | EC₅₀ (µM) | TC₅₀ (µM) | Therapeutic Index |
| Lefamulin | >62.9 | 62.9 | --- |
| TMC353121 | 0.00001 | >1.00 | >100000 |

| | HEp2 cells infected with respiratory syncytial virus (RSV B₁₈₅₃₇) | | |
|---|---|---|---|
| Compound | EC₅₀ (µM) | TC₅₀ (µM) | Therapeutic Index |
| Lefamulin | 17.7 | 71.1 | 4.02 |
| TMC353121 | 0.00001 | >1.00 | >100000 |

### Example 6

**Objective:** The assay measured the inhibition of virus-induced cytopathic effects (CPE) and cell viability during replication of Measles virus strain Edmonston in HeLa cells.

**Method:** HeLa cells were seeded in 96-well flat-bottom tissue culture plates (at a density of 5 x 10³ cells per well) and allowed to adhere overnight. Thereafter, diluted test compounds (Lefamulin as acetate, Ribavirin for control) were added to the plate and incubated for 4 hours prior to addition of the virus. Virus was added diluted to a pre-determined titer to yield 85-95% cell killing at 6 days post-infection (1:50 dilution, MOI of 0.008).

Cell viability determination and calculation of EC₅₀ and TC₅₀ were performed as described in Examples 1 and 3.

### Results:

The antiviral efficacy and cellular toxicity data are summarized in the Table below. Ribavirin was evaluated as control compound in parallel to Lefamulin and yielded an EC₅₀ value of 1.88 µg/mL. Surprisingly, Lefamulin yielded an even lower EC₅₀ value of 0.89 µM with a high calculated TI of 81.7.

| Compound | HeLa Cells infected with Measles virus (strain Edmonston) | | |
|---|---|---|---|
| | EC₅₀ (µM) | TC₅₀ (µM) | Therapeutic Index |
| Lefamulin | 0.89 | 72.7 | 81.7 |
| Ribavirin (µg/mL) | 1.88 | 21.6 | 11.5 |

### Example 7

**Objective:** The assay measured the inhibition of virus-induced cytopathic effects (CPE) and cell viability during replication of Dengue virus strain DENV2_{New Guinea} in Huh7 cells.

**Method:** Huh7 cells were seeded in 96-well flat-bottom tissue culture plates (at a density of 5 x 10³ cells per well) and allowed to adhere overnight. Thereafter, diluted test compounds (Lefamulin as acetate, Ribavirin for control) were added to the plate and incubated for 4 hours prior to addition of the virus. The Dengue virus strain DENV2_{New Guinea} was obtained from ATCC (VR-1584) and was grown in Rhesus monkey kidney cells for the production of stock virus pools. Virus was added diluted to a pre-determined titer to yield 85-95% cell killing at 6 days post-infection (MOI of 0.001).

Cell viability determination as well as EC₅₀ and TC₅₀ calculations were performed as described in Examples 1 and 3.

### Results:

The antiviral efficacy and cellular toxicity data are summarized in the Table below. Ribavirin was evaluated as control compound in parallel to Lefamulin and yielded an EC₅₀ value of 4.73 µg/mL. Lefamulin yielded an EC₅₀ value of 6.79 µM. Ribavirin and Lefamulin both showed a certain cytotoxicity for this specific cell line at concentrations of 48.5 µg/mL and 23.3 µM, respectively.

| Compound | Huh7 Cells infected with Dengue virus (strain DENV2_{New Guinea}) | | |
|---|---|---|---|
| | EC₅₀ (µM) | TC₅₀ (µM) | Therapeutic Index |
| Lefamulin | 6.79 | 23.3 | 3.43 |
| Ribavirin (µg/mL) | 4.73 | 48.5 | 10.3 |

### Example 8

**Objective:** The assay measured the inhibition of virus-induced cytopathic effects (CPE) and cell viability during replication of Zika virus strain ZIK V_{PRVABC59} in Huh7 cells following a 4 hour cell pretreatment.

**Method:** Huh7 cells were seeded in 96-well flat-bottom tissue culture plates (at a density of 5 x 10³ cells per well) and allowed to adhere overnight. Thereafter, diluted test compounds (Lefamulin as acetate, Sofosbuvir for control) were added to the plate and incubated for 4 hours prior to addition of the virus. The Zika virus strain PRVABC59 obtained from ATCC (catalog VR-1843) was obtained from ATCC (VR-1584) and was grown in Rhesus monkey kidney cells for the production of stock virus pools. Virus was added diluted to a pre-determined titer to yield 85-95% cell killing at 6 days post-infection (MOI of 0.001).

Cell viability determination as well as EC₅₀ and TC₅₀ calculations were performed as described in Examples 1 and 3.

### Results:

The antiviral efficacy and cellular toxicity data are summarized in the Table below. The control compound Sofosbuvir was evaluated in parallel to Lefamulin and yielded an EC₅₀ value of 0.65 µg/mL. Lefamulin yielded an EC₅₀ value of 2.78 µM with a calculated therapeutic index of 8.42.

| Compound | Huh7 Cells infected with Zika virus (strain ZIK V_{PRVABC59}) | | |
|---|---|---|---|
| | EC₅₀ (µM) | TC₅₀ (µM) | Therapeutic Index |
| Lefamulin | 2.78 | 23.4 | 8.42 |
| Sofosbuvir | 0.65 | > 10 | > 15.4 |

### Example 9

**Objective:** The assay measured the inhibition of virus-induced cytopathic effects (CPE) and cell viability during replication of human rhinovirus strain HRV16 strain 11757 in H1-HeLa cells following a 4 hour cell pretreatment.

**Method:** H1-HeLa cells were seeded in 96-well flat-bottom tissue culture plates (at a density of 5 x 10³ cells per well) and allowed to adhere overnight. Thereafter, diluted test compounds (Lefamulin as acetate, Rupintrivir for control) were added to the plate and incubated for 4 hours prior to addition of the virus. The virus HRV16₁₁₇₅₇ was added diluted to a pre-determined titer to yield 85-95% cell killing in the untreated virus control wells (MOI of 0.0005).

Cell viability determination as well as EC₅₀ and TC₅₀ calculations were performed as described in Examples 1 and 3.

### Results:

Rupintrivir, a protease inhibitor developed for treatment of rhinoviruses, was evaluated in parallel and yielded an EC₅₀ value of 4.90 nM. Lefamulin yielded an EC₅₀ value of 9.34 µM with a calculated therapeutic index of 2.58.

| Compound | MDCK Cells infected with rhinovirus HRV16₁₁₇₅₇ | | |
|---|---|---|---|
| | EC₅₀ (µM) | TC₅₀ (µM) | Therapeutic Index |
| Lefamulin | 9.34 | 24.1 | 2.58 |
| Rupintrivir | 0.0049 | > 0.10 | > 20.4 |

### Example 10

**Objective:** The assay measured the inhibition of virus-induced cytopathic effects (CPE) and cell viability during replication of influenza virus strain A/PR/8/34 in MDCK cells following a 4 hour cell pretreatment.

**Method:** MDCK cells were seeded in 96-well flat-bottom tissue culture plates (at a density of 5 x 10³ cells per well) and allowed to adhere overnight. Thereafter, diluted test compounds (Lefamulin as acetate, Oseltamivir for control) were added to the plate and incubated for 4 hours prior to addition of the virus. The influenza virus strain A/PR/8/34 was added diluted to a pre-determined titer to yield 90% cell killing in the untreated virus control wells (MOI of 0.0004).

Cell viability determination as well as EC₅₀ and TC₅₀ calculations were performed as described in Examples 1 and 3.

### Results:

Oseltamivir as established influenza drug was evaluated in parallel and yielded an EC₅₀ value of 0.06 µM. Lefamulin was cytotoxic to MDCK cells at concentrations greater than 23 µM. A maximum inhibition of the influenza mediated CPE by 18.4% was measured at 5 µM Lefamulin. Therefore, and because cytotoxicity was observed at 50 µM, an EC₅₀ could not be determined for Lefamulin. However, an *in vivo* activity was observed in an Influenza infection mouse model (see Example 11 below) using a related mouse adapted Influenza A strain (Influenza A/Puerto Rico/8/34 (H1N1)).

| Compound | MDCK Cells infected with influenza virus (strain A/PR/8/34) | | |
|---|---|---|---|
| | EC₅₀ (µM) | TC₅₀ (µM) | Therapeutic Index |
| Lefamulin | > 23.8 | 23.8 | - |
| Oseltamivir | 0.06 | > 200 | > 333 |

### Example 11

**Objective:** Effects of Lefamulin were investigated in an *in vivo* Influenza virus infection model. In the Influenza virus model, mice were challenged with an influenza A (H1N1) strain adapted to mice.

**Methodology:** Adult female BALB/c mice were randomly allocated to three experimental groups of 15 animals and allowed to acclimatize for one week. Treatments were administered subcutaneously starting at Day -1. The negative control group received a vehicle administered twice per day. Lefamulin was investigated at different dosing. In the low dose regime 35 mg/kg of Lefamulin were administered twice daily (corresponding to a dose of 70 mg/kg/day) from Day -1 to Day 6. In the high dose regime, Lefamulin was administered with a dose of 105/mg/kg/day in three injections until day 3 and was altered from Day 3 on due to administration problems at the injection site. The following doses were 70 mg/kg administered twice daily (corresponding to 140 mg/kg/day). On Day 0, all groups were challenged with influenza A/Puerto Rico/8/34 (H1N1).

During the study, animals were scored daily for clinical signs of influenza virus infection to include abnormal coat condition (piloerection), abnormal posture (hunched), abnormal breathing (rapid and/or irregular breathing rate), reduced mobility, ocular discharged, eye closure and/or survival. The signs of severity of disease were added for the scoring system yielding a maximum possible score of 5. When clinical signs were judged as severe, individual animals were taken out of the study prior to the scheduled end of the study.

At Day 6, lung tissue was dissected, assessed for gross pathology, preserved in fixative and stored for histopathology.

Lung consolidation was scored as follows after macroscopic evaluation: >50% (across all lobes) of field occupied by intra-alveolar edema/haemorrhage; extensive vascular degeneration. Lungs removed and fixated on Day 6 were evaluated microscopically in the histopathology. Four main readouts were evaluated (Bronchial/Bronchiolar Degeneration/Hyperplasia, Broncho-interstitial Inflammation, Alveolar Inflammation/Degeneration, Alveolar Edema/Haemorrhage) and scored yielding a maximum total histopathology score of 16, wherein a lower number indicates less signs of histopathological anomalies.

Lung samples were also processed and stored for Day 3 and Day 6 viral titre. On Day 3 and 6, lungs were collected, homogenised and clarified to determine viral load by TCID₅₀ assay on Madin-Darby Canine Kidney (MDCK) cells.

### Results:

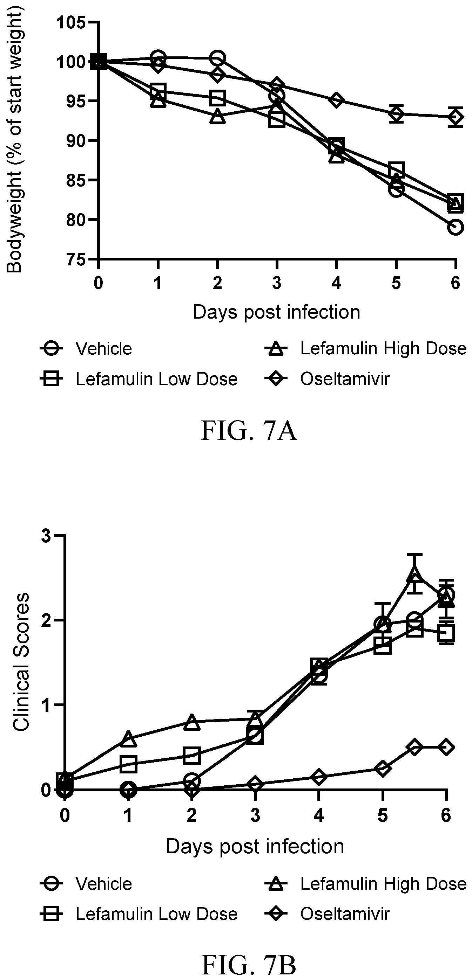

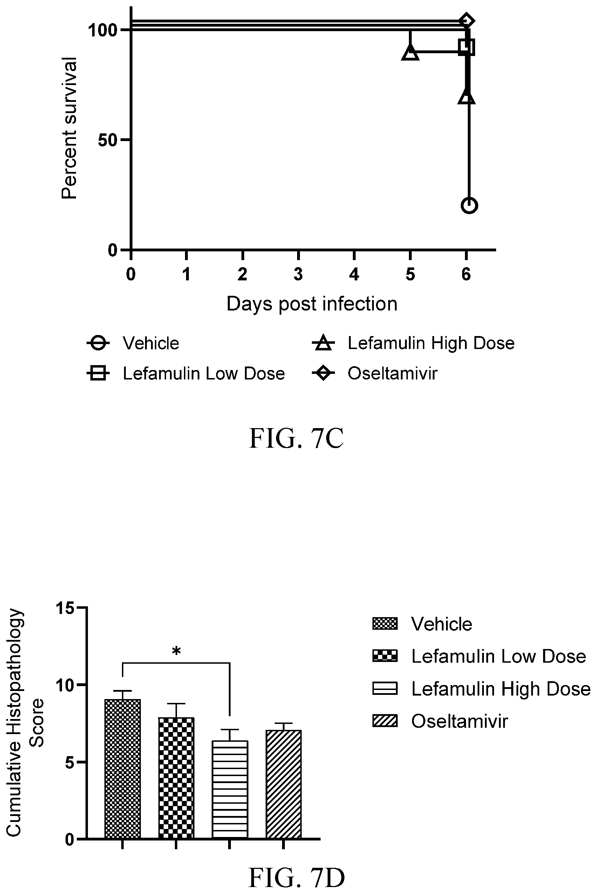

The results of the clinical monitoring are shown in Figures 7A to 7D. The positive control treatment with Oseltamivir worked as expected. A reduction was observed in clinical scores and bodyweight loss for Oseltamivir. Lefamulin did not have a significant effect on bodyweight at the investigated doses (Fig. 7A). At high dose, Lefamulin resulted in an increase in clinical scores and decrease in survival compared to vehicle, which might relate to the local tolerability (SC) issues of the investigated dosage, concentration and formulation (Fig. 7B and C). With the lower dose of Lefamulin, 90 % survival was achieved, whereas only 20 % of the vehicle group survived until day 6 (Fig. 7C).

Moreover, a significant improvement in the macroscopically evaluated lung consolidation was observed for Lefamulin at the low dose. In histopathology, a spectrum of overall individual animal scores (4-13 range) were evident within the specimens examined (Fig. 7D). Lesions were similar to those described within the literature. Increasing severities/distribution area of alveolar pathology were correlated with increases in bronchiolar degeneration/proliferation, broncho-interstitial inflammation and alveola oedema/haemorrhage. Treatment with the high dose of Lefamulin resulted in a significant reduction in bronchial degeneration and alveolar inflammation resulting in an overall significant reduction in histopathology score in this group compared to the vehicle treated control and comparable to Oseltamivir.

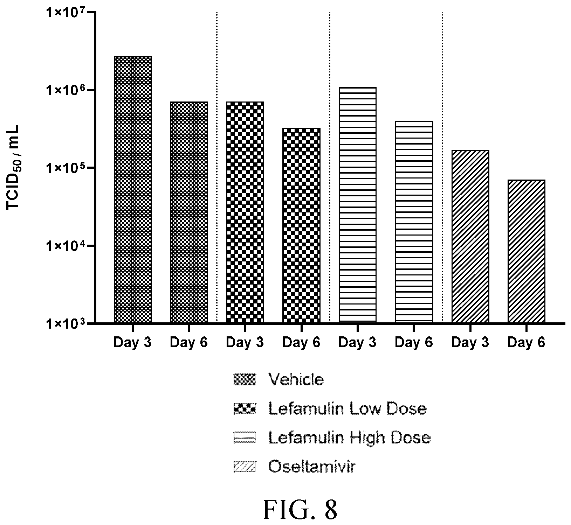

Lung viral titre decreased in all groups between Day 3 and Day 6 (Figure 8). Lefamulin at both doses and Oseltamivir resulted in reduced lung viral titres when compared with the vehicle treated control.

The clinical readout and the reductive effect on the lung viral titer further supports the potential of Lefamulin in the treatment of viral diseases.

### Example 12

**Objective:** The example aims at the synthesis of Lefamulin itaconate as a potential new active pharmaceutical ingredient comprising Lefamulin in protonated form as cation and itaconate as an anion derived from the dicarboxylic itaconic acid.

### Methodology and Result:

To a solution of lefamulin as free base (1 g, 1 eq) in DMF (2 mL) itaconic acid was added (0.5 eq) and stirred at room temperature overnight. The resulting reaction mixture was added dropwise to MTBE. The obtained precipitate was filtered, washed with MTBE and dried under reduced pressure to receive Lefamulin itaconate (1.20 g) in the form of a colorless solid.

¹H-NMR (400 MHz, DMSO-d₆, δ, ppm, characteristic signals, mutilin numbering system described in Berner, H.; Schulz, G.; Schneider H. Tetrahedron 1980, 36, 1807-1811): 6.15 (dd, 1 H, H-19, J=17.6, 11.2 Hz), 5.63 (s, 0.5H, itaconate), 5.55 (d, 1H, H-14, J=8.0 Hz), 5.25-5.00 (m, 2.5H, H-20, itaconate), 3.55 and 3.29 (AB, 2H, H-22, J=15.2 Hz), 3.43 (d, 1H, H-11, J=5.6 Hz), 3.05 (s, 1H, itaconate), 1.37 (s, 3H, CH₃-15), 1.06 (s, 3H, CH₃-18), 0.82 (d, 3H, CH₃-17, J=7.2 Hz), 0.63 (d, 3H, CH₃-16, J=6.8 Hz). MS m/z: 508 [M + H⁺], 552 [M + HCOO⁻].

## Claims

1. A compound of formula (I) 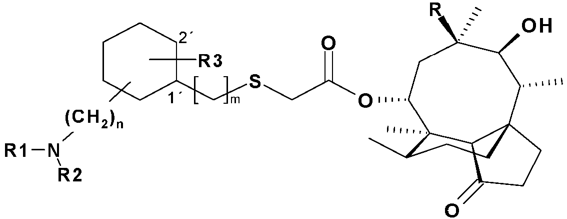 wherein
n is 0 to 4;
m is 0 or 1 with the proviso that the sulphur atom and R₃ are in vicinal position (if m = 0 then R₃ is in position 2', and if m = 1 then R₃ is on position 1 ');
R is ethyl or vinyl;
R₁ is hydrogen or (C₁₋₆)alkyl,
R₂ is hydrogen or
- (C₃₋₆)cycloalkyl, or
- unsubstituted (C₁₋₆)alkyl, or
- (C₁₋₆)alkyl substituted by one or more of
- hydroxy; preferably one or two,
- methoxy,
- halogen,
- (C₃₋₆)cycloalkyl, or
R₁ and R₂ together with the nitrogen atom to which they are attached form a 5 to 7 membered heterocyclic ring containing at least 1 nitrogen atom or 1 nitrogen and 1 additional heteroatom e. g. selected from N or O, or
R₁ is hydroxy and R₂ is formyl;
R₃ is OH, OR₄, a halogen atom, or
R₃ is bound to 2' and represents -O-(CH₂)ₚ-O- with p is 2 or 3;
R₄ is unsubstituted (C₁₋₆)alkyl or (C₃₋₆)cycloalkyl,
or a pharmaceutically acceptable salt or solvate thereof
for the specific use in the treatment or prevention of a viral infection.

2. Compound for use according to claim 1, **characterized in that** the compound is selected from the group consisting of the compounds of formula (II), (III), (IV), (V) and (VI) 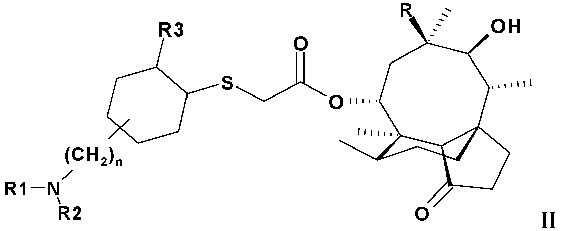 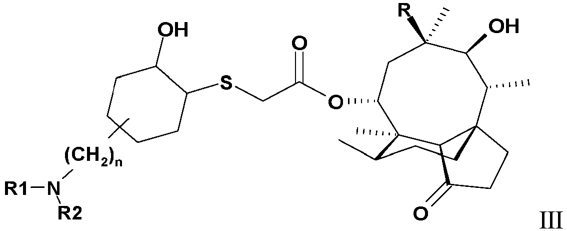 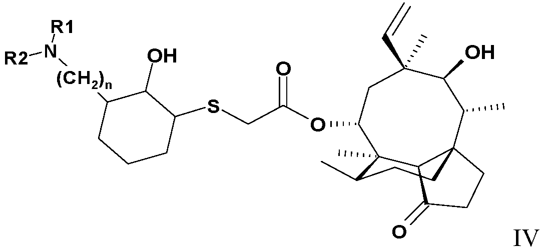 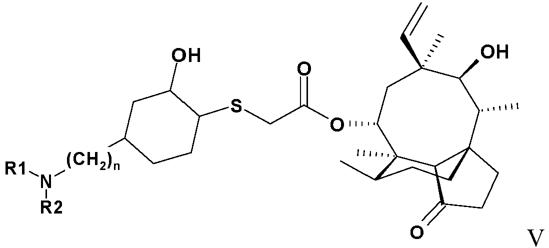 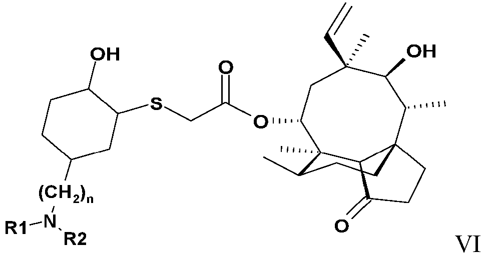 wherein in each case n, R₁ and R₂ are as defined in claim 1.

3. A compound for use according to claim 1 or 2, **characterized in that** the compound is selected from the group consisting of
14-O-{[(1R, 2R, 4R)-4-Amino-2-hydroxy-cyclohexylsulfanyl]-acetyl}-mutilin
14-O-{[(1S, 2S, 4S)-4-Amino-2-hydroxy-cyclohexylsulfanyl]-acetyl}-mutilin
14-O-{[(1R, 2R, 5S)-5-Amino-2-hydroxy-cyclohexylsulfanyl]-acetyl}-mutilin
14-O-{[(1S, 2S, 5R)-5-Amino-2-hydroxy-cyclohexylsulfanyl]-acetyl}-mutilin
14-O-{[(1R, 2R, 4S)-4-Amino-2-hydroxy-cyclohexylsulfanyl]-acetyl}-mutilin and the (1S, 2S, 4R) diastereomer thereof
14-O-{[(1R, 2R, 5R)-5-Amino-2-hydroxy-cyclohexylsulfanyl]-acetyl}-mutilin
14-O-{[(1S, 2S, 5S)-5-Amino-2-hydroxy-cyclohexylsulfanyl]-acetyl}-mutilin
14-O-{[(1R, 2R, 3R)-3-Amino-2-hydroxy-cyclohexylsulfanyl]-acetyl}-mutilin and the (1S, 2S, 3S) diastereomer thereof
14-O-{[(1R, 2R, 4R)-4-Diethylamino-2-hydroxy-cyclohexylsulfanyl]-acetyl}-mutilin and the (1S, 2S, 4S) diastereomer thereof
14-O-{[(1R, 2R, 4R)-4-Ethylamino-2-hydroxy-cyclohexylsulfanyl]-acetyl}-mutilin and the (1S, 2S, 4S) diastereomer thereof
14-O-{[(1R, 2R, 5S)-5-Ethylamino-2-hydroxy-cyclohexylsulfanyl]-acetyl}-mutilin and the (1S, 2S, 5R) diastereomer thereof
14-O-{[(1R, 2R, 5S)-5-Diethylamino-2-hydroxy-cyclohexylsulfanyl]-acetyl}-mutilin and the (1S, 2S, 5R) diastereomer thereof
14-O-{[(1R, 2R, 4S)-4-Diethylamino-2-hydroxy-cyclohexylsulfanyl]-acetyl}-mutilin and the (1S, 2S, 4R) diastereomer thereof
14-O-{[(1R, 2R, 5R)-5-Diethylamino-2-hydroxy-cyclohexylsulfanyl]-acetyl}-mutilin and the (1S, 2S, 5S) diastereomer thereof
14-O-{[(1R, 2R, 3R)-3-Ethylamino-2-hydroxy-cyclohexylsulfanyl]-acetyl}-mutilin and the (1S, 2S, 3S) diastereomer thereof
14-O-{[(1R, 2R, 3R)-3-Diethylamino-2-hydroxy-cyclohexylsulfanyl]-acetyl}-mutilin and the (1S, 2S, 3S) diastereomer thereof
14-O-{[(1R, 2R, 4S)-4-(Formyl-hydroxy-amino)-2-hydroxy-cyclohexylsulfanyl]-acetyl}-mutilin and the (1S, 2S, 4R) diastereomer thereof
14-O-{[(1R, 2R, 5S)-5-(Formyl-hydroxy-amino)-2-hydroxy-cyclohexylsulfanyl]-acetyl}-mutilin and the (1S, 2S, 5R) diastereomer thereof
14-O-{[(1R, 2R, 3R/S)-3-(Formyl-hydroxy-amino)-2-hydroxy-cyclohexylsulfanyl]-acetyl}-mutilin and the (1S, 2S, 3R/S) diastereomer thereof
14-O-{[(1R, 2R, 5S)- 2-Hydroxy-5-methylamino-cyclohexylsulfanyl]-acetyl}-mutilin and the (1S, 2S, 5R) diastereomer thereof
14-O-{[(1R, 2R, 5S)-5-Allylamino-2-hydroxy-cyclohexylsulfanyl]-acetyl}-mutilin and the (1S, 2S, 5R) diastereomer thereof
14-O-{[(1R, 2R, 5S)-2-Hydroxy-5-(2-methoxy-ethylamino)-cyclohexylsulfanyl]-acetyl}-mutilin and the (1S, 2S, 5R) diastereomer thereof
14-O-{[(1R, 2R, 4R*)-2-Hydroxy-4-(2-hydroxy-ethylamino)-cyclohexylsulfanyl]-acetyl}-mutilin and the (1S, 2S, 4S*) diastereomer thereof
14-O-{[(1R, 2R, 4R*)-4-Cyclohexylamino-2-hydroxy-cyclohexylsulfanyl]-acetyl}-mutilin and the (1S, 2S, 4S*) diastereomer thereof
14-O-{[(1R, 2R, 4R*)-4-Cyclopropylamino-2-hydroxy-cyclohexylsulfanyl]-acetyl}-mutilin and the (1S, 2S, 4S*) diastereomer thereof
14-O-{[(1R, 2R, 5S*)-4-Cyclopropylamino-2-hydroxy-cyclohexylsulfanyl]-acetyl}-mutilin and the (1S, 2S, 5R*) diastereomer thereof
14-O-{[(1R, 2R, 4S*)-4-Cyclopropylamino-2-hydroxy-cyclohexylsulfanyl]-acetyl}-mutilin and the (1S, 2S, 4R*) diastereomer thereof
14-O-{[(1R, 2R, 5R*)-2-Hydroxy-5-morpholin-4-yl-cyclohexylsulfanyl]-acetyl}-mutilin and the (1S, 2S, 5S*) diastereomer thereof
14-O-{[(1R, 2R, 5S*)-2-Hydroxy-5-morpholin-4-yl-cyclohexylsulfanyl]-acetyl}-mutilin and the (1S, 2S, 5R*) diastereomer thereof
14-O-{[(1R, 2R, 5S)-5-Amino-2-hydroxy-cyclohexylsulfanyl]-acetyl}-19,20-dihydro-mutilin and the (1S, 2S, 5R) diastereomer thereof
14-O-{[(1R, 2R, 5S)-5-Ethylamino-2-hydroxy-cyclohexylsulfanyl]-acetyl}-19,20-dihydro-mutilin and the (1S, 2S, 5R) diastereomer thereof
14-O-{[(1R, 2R, 5R)-5-Amino-2-hydroxy-cyclohexylsulfanyl]-acetyl}-19,20-dihydro-mutilin and the (1S, 2S, 5S) diastereomer thereof
14-O-{[(1R, 2R)-4-Aminomethyl-2-hydroxy-cyclohexylsulfanyl]-acetyl}-mutilin and the (1S, 2S) diastereomers thereof
14-O-{[5-Amino-2-chloro-cyclohexylsulfanyl]-acetyl}-mutilin
14-O-{[4-Amino-2-chloro-cyclohexylsulfanyl]-acetyl}-mutilin 14-O-[(4-Amino-1-hydroxy-cyclohexylmethylsulfanyl)-acetyl]-mutilin
14-O-{[(1R, 2R)-2-Hydroxy-5-(3-methylamino-propyl)-cyclohexylsulfanyl]-acetyl}-mutilin and the (1S, 2S) diastereomer thereof
14-O-{[(1R, 2R)-2-Hydroxy-4-(3-methylamino-propyl)-cyclohexylsulfanyl]-acetyl}-mutilin and the (1S, 2S) diastereomer thereof
14-O-{[(1R, 2R)-5-(3-Amino-propyl)-2-hydroxy-cyclohexylsulfanyl]-acetyl}-mutilin and the (1S, 2S) diastereomer thereof
14-O-{[(1R, 2R)-4-(3-Amino-propyl)-2-hydroxy-cyclohexylsulfanyl]-acetyl}-mutilin and the (1S, 2S) diastereomer thereof
14-O-{[(6R, 8R)-8-Amino-1,4-dioxa-spiro[4.5]dec-6-ylsulfanyl]-acetyl}-mutilin and the (6S, 8S) diastereomer thereof
14-O-{[4-Amino-2-methoxy-cyclohexylsulfanyl]-acetyl}-mutilin and
14-O-{[5-Amino-2-methoxy-cyclohexylsulfanyl]-acetyl}-mutilin.

4. A compound for use according to any of claims 1 to 3, **characterized in that** the compound is Lefamulin.

5. A compound for use according to any of claims 1 to 4, **characterized in that** the compound is in the form of a salt and/or a solvate.

6. A compound for use according to any of claims 1 to 5, **characterized in that** the compound is Lefamulin in the form as Lefamulin acetate salt.

7. A compound for use according to any of the preceding claims, **characterized in that** the viral infection is a respiratory disease.

8. A compound for use according to any of the preceding claims, **characterized in that** the viral infection is an acute respiratory syndrome, such as Influenza, Severe Acute Respiratory Syndrome (SARS), Middle East Respiratory Syndrome (MERS) or COVID-19.

9. A compound for use according to any of the preceeding claims, **characterized in that** the virus mediating the viral infection is a positive- or negative-sense single-stranded RNA virus, preferably the virus is selected from the group consisting of
- Coronaviridae including in particular human coronavirus,
- Paramyxoviridae including in particular Paramyxovirinae, such as Measles virus, and Pneumovirinae, such as Respiratory Syncytial Virus,
- Orthomyxoviridae including in particular Influenza virus,
- Flaviviridae including in particular Dengue virus and Zika virus, and
- Picornaviridae including in particular Rhinovirus.

10. A compound for use according to any of the preceeding claims, **characterized in that** the viral infection is an airborne disease.

## Patentansprüche

1. Verbindung der Formel (I) 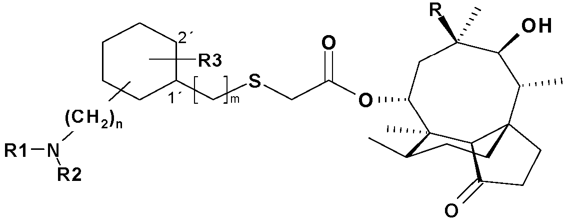 worin
n 0 bis 4 ist;
m 0 oder 1 ist, unter der Voraussetzung, dass sich das Schwefelatom und R₃ in benachbarter Position sind (wenn m = 0, dann ist R₃ in Position 2', und wenn m = 1, dann ist R₃ in Position 1');
R Ethyl oder Vinyl ist;
R₁ Wasserstoff oder (C₁₋₆)-Alkyl ist,
R₂ Wasserstoff ist oder
- (C₃₋₆)-Cycloalkyl oder
- nicht substituiertes (C₁₋₆)-Alkyl, oder
- (C₁₋₆)-Alkyl, substituiert durch ein oder mehrere
- Hydroxy; vorzugsweise ein oder zwei,
- Methoxy,
- Halogen,
- (C₃₋₆)-Cycloalkyl oder
R₁ und R₂ bilden gemeinsam mit dem Stickstoffatom, an welchem sie gebunden sind, einen 5- bis 7-gliedrigen heterocyclischen Ring, der mindestens 1 Stickstoffatom oder 1 Stickstoff und 1 zusätzliches Heteroatom enthält, z. B. ausgewählt aus N oder O, oder
R₁ Hydroxy ist und R₂ Formyl ist;
R₃ OH, OR₄, ein Halogenatom ist, oder
R₃ an 2' gebunden ist und -O-(CH₂)ₚ-O- ist, mit p =2 oder 3;
R₄ nicht substituiertes (C₁₋₆)-Alkyl oder (C₃₋₆)-Cycloalkyl ist,
oder ein pharmazeutisch akzeptables Salz oder Solvat davon
zur spezifischen Verwendung bei der Behandlung oder Vorbeugung einer viralen Infektion.

2. Verbindung zur Verwendung nach Anspruch 1, **dadurch gekennzeichnet, dass** die Verbindung ausgewählt ist aus der Gruppe bestehend aus den Verbindungen der Formel (II), (III), (IV), (V) und (VI) 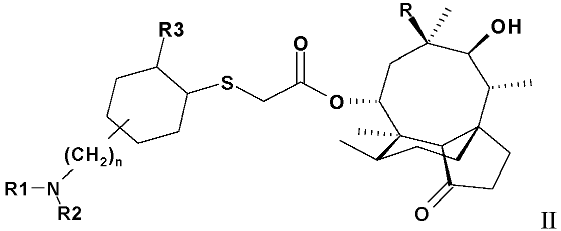 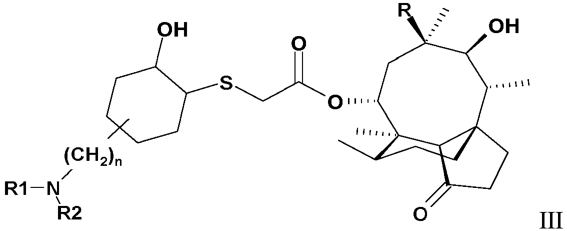 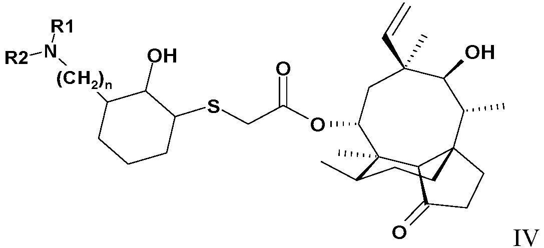 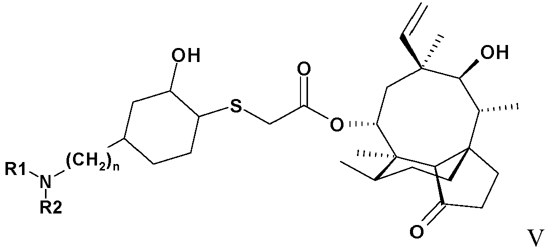 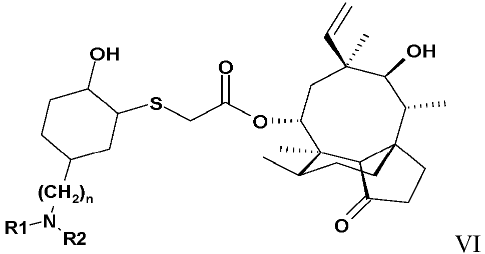 worin jeweils n, R₁ und R₂ wie in Anspruch 1 definiert sind.

3. Verbindung zur Verwendung gemäß Anspruch 1 oder 2, **dadurch gekennzeichnet, dass** die Verbindung ausgewählt ist aus der Gruppe bestehend aus
14-O-{[(1R, 2R, 4R)-4-Amino-2-hydroxy-cyclohexylsulfanyl]-acetyl}-mutilin
14-O-{[(1S, 2S, 4S)-4-Amino-2-hydroxy-cyclohexylsulfanyl]-acetyl}-mutilin
14-O-{[(1R, 2R, 5S)-5-Amino-2-hydroxy-cyclohexylsulfanyl]-acetyl}-mutilin
14-O-{[(1S, 2S, 5R)-5-Amino-2-hydroxy-cyclohexylsulfanyl]-acetyl}-mutilin
14-O-{[(1R, 2R, 4S)-4-Amino-2-hydroxy-cyclohexylsulfanyl]-acetyl}-mutilin und das (1S, 2S, 4R)-Diastereomer davon
14-O-{[(1R, 2R, 5R)-5-Amino-2-hydroxy-cyclohexylsulfanyl]-acetyl}-mutilin
14-O-{[(1S, 2S, 5S)-5-Amino-2-hydroxy-cyclohexylsulfanyl]-acetyl}-mutilin
14-O-{[(1R, 2R, 3R)-3-Amino-2-hydroxy-cyclohexylsulfanyl]-acetyl}-mutilin und das (1S, 2S, 3S)-Diastereomer davon
14-O-{[(1R, 2R, 4R)-4-Diethylamino-2-hydroxy-cyclohexylsulfanyl]-acetyl}-mutilin und das (1S, 2S, 4S)-Diastereomer davon
14-O-{[(1R, 2R, 4R)-4-Ethylamino-2-hydroxy-cyclohexylsulfanyl]-acetyl}-Mutilin und das (1S, 2S, 4S)-Diastereomer davon
14-O-{[(1R, 2R, 5S)-5-Ethylamino-2-hydroxy-cyclohexylsulfanyl]-acetyl}-mutilin und das (1S, 2S, 5R)-Diastereomer davon
14-O-{[(1R, 2R, 5S)-5-Diethylamino-2-hydroxy-cyclohexylsulfanyl]-acetyl}-mutilin und das (1S, 2S, 5R)-Diastereomer davon
14-O-{[(1R, 2R, 4S)-4-Diethylamino-2-hydroxy-cyclohexylsulfanyl]-acetyl}-mutilin und das (1S, 2S, 4R)-Diastereomer davon
14-O-{[(1R, 2R, 5R)-5-Diethylamino-2-hydroxy-cyclohexylsulfanyl]-acetyl}-mutilin und das (1S, 2S, 5S)-Diastereomer
14-O-{[(1R, 2R, 3R)-3-Ethylamino-2-hydroxy-cyclohexylsulfanyl]-acetyl}-Mutilin und das (1S, 2S, 3S)-Diastereomer davon
14-O-{[(1R, 2R, 3R)-3-Diethylamino-2-hydroxy-cyclohexylsulfanyl]-acetyl}-mutilin und das (1S, 2S, 3S)-Diastereomer davon
14-O-{[(1R, 2R, 4S)-4-(Formyl-hydroxy-amino)-2-hydroxy-cyclohexylsulfanyl]-acetyl}-mutilin und das (1S, 2S, 4R)-Diastereomer davon
14-O-{[(1R, 2R, 5S)-5-(Formyl-hydroxy-amino)-2-hydroxy-cyclohexylsulfanyl]-acetyl}-mutilin und das (1S, 2S, 5R)-Diastereomer davon
14-O-{[(1R, 2R, 3R/S)-3-(Formyl-hydroxy-amino)-2-hydroxy-cyclohexylsulfanyl]-acetyl}-mutilin und das (1S, 2S, 3R/S)-Diastereomer davon
14-O-{[(1R, 2R, 5S)- 2-Hydroxy-5-methylamino-cyclohexylsulfanyl]-acetyl}-mutilin und das (1S, 2S, 5R)-Diastereomer davon
14-O-{[(1R, 2R, 5S)-5-Allylamino-2-hydroxy-cyclohexylsulfanyl]-acetyl}-mutilin und das (1S, 2S, 5R)-Diastereomer davon
14-O-{[(1R, 2R, 5S)-2-Hydroxy-5-(2-methoxy-ethylamino)-cyclohexylsulfanyl]-acetyl}-mutilin und das (1S, 2S, 5R)-Diastereomer
14-O-{[(1R, 2R, 4R*)-2-Hydroxy-4-(2-hydroxy-ethylamino)-cyclohexylsulfanyl]-acetyl}-mutilin und das (1S, 2S, 4S*)-Diastereomer davon
14-O-{[(1R, 2R, 4R*)-4-Cyclohexylamino-2-hydroxy-cyclohexylsulfanyl]-acetyl}-mutilin und das (1S, 2S, 4S*)-Diastereomer davon
14-O-{[(1R, 2R, 4R*)-4-Cyclopropylamino-2-hydroxy-cyclohexylsulfanyl]-acetyl}-mutilin und das (1S, 2S, 4S*)-Diastereomer davon
14-O-{[(1R, 2R, 5S*)-4-Cyclopropylamino-2-hydroxy-cyclohexylsulfanyl]-acetyl}-mutilin und das (1S, 2S, 5R*)-Diastereomer davon
14-O-{[(1R, 2R, 4S*)-4-Cyclopropylamino-2-hydroxy-cyclohexylsulfanyl]-acetyl}-mutilin und das (1S, 2S, 4R*)-Diastereomer davon
14-O-{[(1R, 2R, 5R*)-2-Hydroxy-5-morpholin-4-yl-cyclohexylsulfanyl]-acetyl}-mutilin und das (1S, 2S, SS*)-Diastereomer davon
14-O-{[(1R, 2R, 5S*)-2-Hydroxy-5-morpholin-4-yl-cyclohexylsulfanyl]-acetyl}-mutilin und das (1S, 2S, 5R*)-Diastereomer davon
14-O-{[(1R, 2R, 5S)-5-Amino-2-hydroxy-cyclohexylsulfanyl]-acetyl}-19,20-dihydro-mutilin und das (1S, 2S, 5R)-Diastereomer davon
14-O-{[(1R, 2R, 5S)-5-Ethylamino-2-hydroxy-cyclohexylsulfanyl]-acetyl}-19,20-dihydro-mutilin und das (1S, 2S, 5R)-Diastereomer davon
14-O-{[(1R, 2R, 5R)-5-Amino-2-hydroxy-cyclohexylsulfanyl]-acetyl}-19,20-dihydro-mutilin und das (1S, 2S, SS)-Diastereomer davon
14-O-{[(1R, 2R)-4-Aminomethyl-2-hydroxy-cyclohexylsulfanyl]-acetyl}-mutilin und die (1S, 2S)-Diastereomere davon
14-O-{[5-Amino-2-chlor-cyclohexylsulfanyl]-acetyl}-mutilin
14-O-{[4-Amino-2-chlor-cyclohexylsulfanyl]-acetyl}-mutilin
14-O-[(4-Amino-1-hydroxy-cyclohexylmethylsulfanyl)-acetyl]-mutilin
14-O-{[(1R, 2R)-2-Hydroxy-5-(3-methylamino-propyl)-cyclohexylsulfanyl]-acetyl}-mutilin und das (1S, 2S)-Diastereomer davon
14-O-{[(1R, 2R)-2-Hydroxy-4-(3-methylamino-propyl)-cyclohexylsulfanyl]-acetyl}-mutilin und das (1S, 2S)-Diastereomer davon
14-O-{[(1R, 2R)-5-(3-Amino-propyl)-2-hydroxy-cyclohexylsulfanyl]-acetyl}-mutilin und das (1S, 2S)-Diastereomer davon
14-O-{[(1R, 2R)-4-(3-Amino-propyl)-2-hydroxy-cyclohexylsulfanyl]-acetyl}-mutilin und das (1S, 2S)-Diastereomer davon
14-O-{[(6R, 8R)-8-Amino-1,4-dioxa-spiro[4.5]dec-6-ylsulfanyl]-acetyl}-mutilin und das (6S, 8S)-Diastereomer davon
14-O-{[4-Amino-2-methoxy-cyclohexylsulfanyl]-acetyl}-mutilin und
14-O-{[5-Amino-2-methoxy-cyclohexylsulfanyl]-acetyl}-mutilin.

4. Verbindung zur Verwendung nach einem der Ansprüche 1 bis 3, **dadurch gekennzeichnet, dass** die Verbindung Lefamulin ist.

5. Verbindung zur Verwendung nach einem der Ansprüche 1 bis 4, **dadurch gekennzeichnet, dass** die Verbindung in Form eines Salzes und/oder eines Solvats vorliegt.

6. Verbindung zur Verwendung nach einem der Ansprüche 1 bis 5, **dadurch gekennzeichnet, dass** die Verbindung Lefamulin in der Form als Lefamulin-AcetatSalz vorliegt.

7. Verbindung zur Verwendung nach einem der vorhergehenden Ansprüche, **dadurch gekennzeichnet, dass** die virale Infektion eine Atemwegserkrankung ist.

8. Verbindung zur Verwendung nach einem der vorhergehenden Ansprüche, **dadurch gekennzeichnet, dass** die virale Infektion ein akutes respiratorisches Syndrom ist, wie Influenza, Schweres Akutes Respiratorisches Syndrom (SARS), Middle East Respiratory Syndrom (MERS) oder COVID-19.

9. Verbindung zur Verwendung nach einem der vorangehenden Ansprüche, **dadurch gekennzeichnet, dass** das Virus, das die virale Infektion vermittelt, ein einzelsträngiges RNA-Virus positiver oder negativer Polarität ist, wobei das Virus vorzugsweise ausgewählt ist aus der Gruppe bestehend aus
- Coronaviridae, einschließlich insbesondere das humane Coronavirus
- Paramyxoviridae, einschließlich insbesondere Paramyxovirinae, wie das Masernvirus, und Pneumovirinae, wie das Respiratorische Synzytial-Virus,
- Orthomyxoviridae, einschließlich insbesondere das Influenzavirus,
- Flaviviridae, einschließlich insbesondere Dengue-Virus und Zika-Virus, und
- Picornaviridae, einschließlich insbesondere das Rhinovirus.

10. Verbindung zur Verwendung nach einem der vorangehenden Ansprüche, **dadurch gekennzeichnet, dass** die virale Infektion eine durch die Luft übertragene Krankheit ist.

## Revendications

1. Composé de formule (I) 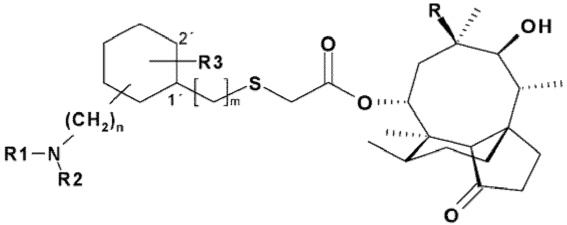 dans lequel
n vaut 0 à 4;
m vaut 0 ou 1 à condition que l'atome du soufre et R₃ soient en position vicinale (si m = 0 alors R₃ est en position 2', et si m = 1 alors R₃ est sur la position 1');
R est un éthyle ou un vinyle;
R₁ est un hydrogène ou un alkyle en (C₁₋₆),
R₂ est un hydrogène ou
- un cycloalkyle en (C₃₋₆), ou
- un alkyle en (C₁₋₆) non substitué, ou
- un alkyle en (C₁₋₆) substitué par un ou plusieurs parmi
- un hydroxy; de préférence un ou deux,
- un méthoxy,
- un halogène,
- un cycloalkyle en (C₃₋₆), ou
R₁ et R₂ conjointement à l'atome d'azote auquel ils sont fixés forment un cycle hétérocyclique de 5 à 7 chaînons contenant au moins 1 atome d'azote ou 1 azote et 1 hétéroatome supplémentaire choisi par exemple parmi N ou O, ou
R₁ est un hydroxy et R₂ est un formyle;
R₃ est OH, OR₄, un atome d'halogène, ou
R₃ est lié en 2', et représente -O-(CH₂)ₚ-O- avec p vaut 2 ou 3;
R₄ est un alkyle en (C₁₋₆) non substitué ou un cycloalkyle en (C₃₋₆),
ou un sel ou solvate pharmaceutiquement acceptable de celui-ci,
pour l'utilisation spécifique dans le traitement ou la prévention d'une infection virale.

2. Composé destiné à être utilisé selon la revendication 1, **caractérisé en ce que** le composé est choisi dans le groupe constitué par les composés de formule (II), (III), (IV), (V) et (VI) 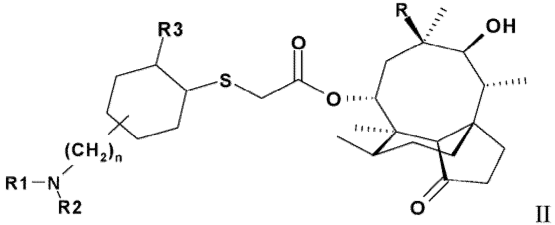 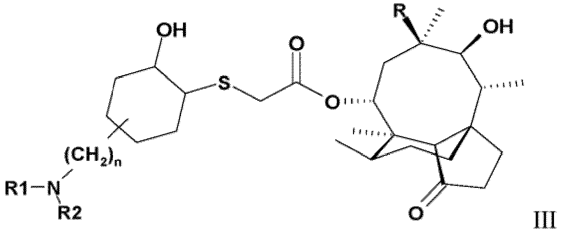 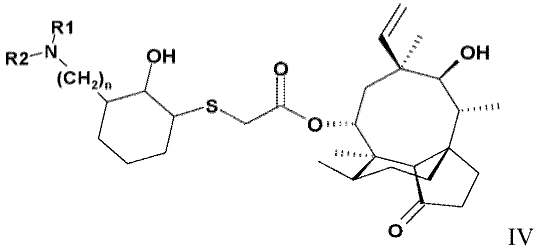 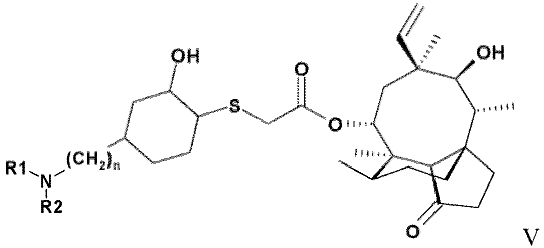 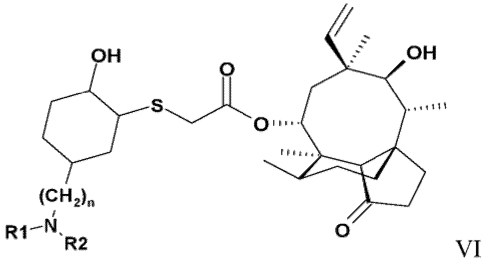 dans lequel n, R₁ et R₂ sont chacun tels que définis dans la revendication 1.

3. Composé destiné à être utilisé selon la revendication 1 ou 2, **caractérisé en ce que** le composé est choisi dans le groupe constitué par
la 14-O-{[(1R,2R,4R)-4-amino-2-hydroxy-cyclohexylsulfanyl]-acétyl}-mutiline
la 14-O-{[(1S,2S,4S)-4-amino-2-hydroxy-cyclohexylsulfanyl]-acétyl}-mutiline
la 14-O-{[(1R,2R,5S)-5-amino-2-hydroxy-cyclohexylsulfanyl]-acétyl}-mutiline
la 14-O-{[(1S,2S,5R)-5-amino-2-hydroxy-cyclohexylsulfanyl]-acétyl}-mutiline
la 14-O-{[(1R,2R,4S)-4-amino-2-hydroxy-cyclohexylsulfanyl]-acétyl}-mutiline et le diastéréomère (1S,2S,4R) de celle-ci
la 14-O-{[(1R,2R,5R)-5-amino-2-hydroxy-cyclohexylsulfanyl]-acétyl}-mutiline
la 14-O-{[(1S,2S,5S)-5-amino-2-hydroxy-cyclohexylsulfanyl]-acétyl}-mutiline
la 14-O-{[(1R,2R,3R)-3-amino-2-hydroxy-cyclohexylsulfanyl]-acétyl}-mutiline et le diastéréomère (1S,2S,3S) de celle-ci
la 14-O-{[(1R,2R,4R)-4-diéthylamino-2-hydroxy-cyclohexylsulfanyl]-acétyl}-mutiline et le diastéréomère (1S,2S,4S) de celle-ci
la 14-O-{[(1R,2R,4R)-4-éthylamino-2-hydroxy-cyclohexylsulfanyl]-acétyl}-mutiline et le diastéréomère (1S,2S,4S) de celle-ci
la 14-O-{[(1R,2R,5S)-5-éthylamino-2-hydroxy-cyclohexylsulfanyl]-acétyl}-mutiline et le diastéréomère (1S,2S,5R) de celle-ci
la 14-O-{[(1R,2R,5S)-5-diéthylamino-2-hydroxy-cyclohexylsulfanyl]-acétyl}-mutiline et le diastéréomère (1S,2S,5R) de celle-ci
la 14-O-{[(1R,2R,4S)-4-diéthylamino-2-hydroxy-cyclohexylsulfanyl]-acétyl}-mutiline et le diastéréomère (1S,2S,4R) de celle-ci
la 14-O-{[(1R,2R,5R)-5-diéthylamino-2-hydroxy-cyclohexylsulfanyl]-acétyl}-mutiline et le diastéréomère (1S,2S,5S) de celle-ci
la 14-O-{[(1R,2R,3R)-3-éthylamino-2-hydroxy-cyclohexylsulfanyl]-acétyl}-mutiline et le diastéréomère (1S,2S,3S) de celle-ci
la 14-O-{[(1R,2R,3R)-3-diéthylamino-2-hydroxy-cyclohexylsulfanyl]-acétyl}-mutiline et le diastéréomère (1S,2S,3S) de celle-ci
la 14-O-{[(1R,2R,4S)-4-(formyl-hydroxy-amino)-2-hydroxy-cyclohexylsulfanyl]-acétyl}-mutiline et le diastéréomère (1S,2S,4R) de celle-ci
la 14-O-{[(1R,2R,5S)-5-(formyl-hydroxy-amino)-2-hydroxy-cyclohexylsulfanyl]-acétyl}-mutiline et le diastéréomère (1S,2S,5R) de celle-ci
la 14-O-{[(1R,2R,3R/S)-3-(formyl-hydroxy-amino)-2-hydroxy-cyclohexylsulfanyl]-acétyl}-mutiline et le diastéréomère (1S,2S,3R/S) de celle-ci
la 14-O-{[(1R,2R,5S)-2-hydroxy-5-méthylamino-cyclohexylsulfanyl]-acétyl}-mutiline et le diastéréomère (1S,2S,5R) de celle-ci
la 14-O-{[(1R,2R,5S)-5-allylamino-2-hydroxy-cyclohexylsulfanyl]-acétyl}-mutiline et le diastéréomère (1S,2S,5R) de celle-ci
la 14-O-{[(1R,2R,5S)-2-hydroxy-5-(2-méthoxy-éthylamino)-cyclohexylsulfanyl]-acétyl}-mutiline et le diastéréomère (1S,2S,5R) de celle-ci
la 14-O-{[(1R,2R,4R*)-2-hydroxy-4-(2-hydroxy-éthylamino)-cyclohexylsulfanyl]-acétyl}-mutiline et le diastéréomère (1S,2S,4S*) de celle-ci
la 14-O-{[(1R,2R,4R*)-4-cyclohexylamino-2-hydroxy-cyclohexylsulfanyl]-acétyl}-mutiline et le diastéréomère (1S,2S,4S*) de celle-ci
la 14-O-{[(1R,2R,4R*)-4-cyclopropylamino-2-hydroxy-cyclohexylsulfanyl]-acétyl}-mutiline et le diastéréomère (1S,2S,4S*) de celle-ci
la 14-O-{[(1R,2R,5S*)-4-cyclopropylamino-2-hydroxy-cyclohexylsulfanyl]-acétyl}-mutiline et le diastéréomère (1S,2S,5R*) de celle-ci
la 14-O-{[(1R,2R,4S*)-4-cyclopropylamino-2-hydroxy-cyclohexylsulfanyl]-acétyl}-mutiline et le diastéréomère (1S,2S,4R*) de celle-ci
la 14-O-{[(1R,2R,5R*)-2-hydroxy-5-morpholin-4-yl-cyclohexylsulfanyl]-acétyl}-mutiline et le diastéréomère (1S,2S,5S*) de celle-ci
la 14-O-{[(1R,2R,5S*)-2-hydroxy-5-morpholin-4-yl-cyclohexylsulfanyl]-acétyl}-mutiline et le diastéréomère (1S,2S,5R*) de celle-ci
la 14-O-{[(1R,2R,5S)-5-amino-2-hydroxy-cyclohexylsulfanyl]-acétyl}-19,20-dihydro-mutiline et le diastéréomère (1S,2S,5R) de celle-ci
la 14-O-{[(1R,2R,5S)-5-éthylamino-2-hydroxy-cyclohexylsulfanyl]-acétyl}-19,20-dihydro-mutiline et le diastéréomère (1S,2S,5R) de celle-ci
la 14-O-{[(1R,2R,5R)-5-amino-2-hydroxy-cyclohexylsulfanyl]-acétyl}-19,20-dihydro-mutiline et le diastéréomère (1S,2S,5S) de celle-ci
la 14-O-{[(1R,2R)-4-aminométhyl-2-hydroxy-cyclohexylsulfanyl]-acétyl}-mutiline et les diastéréomères (1S,2S) de celle-ci
la 14-O-{[5-amino-2-chloro-cyclohexylsulfanyl]-acétyl}-mutiline
la 14-O-{[4-amino-2-chloro-cyclohexylsulfanyl]-acétyl}-mutiline
la 14-O-[(4-amino-1-hydroxy-cyclohexylméthylsulfanyl)-acétyl]-mutiline
la 14-O-{[(1R,2R)-2-hydroxy-5-(3-méthylamino-propyl)-cyclohexylsulfanyl]-acétyl}-mutiline et le diastéréomère (1S,2S) de celle-ci
la 14-O-{[(1R,2R)-2-hydroxy-4-(3-méthylamino-propyl)-cyclohexylsulfanyl]-acétyl}-mutiline et le diastéréomère (1S,2S) de celle-ci
la 14-O-{[(1R,2R)-5-(3-amino-propyl)-2-hydroxy-cyclohexylsulfanyl]-acétyl}-mutiline et le diastéréomère (1S,2S) de celle-ci
la 14-O-{[(1R,2R)-4-(3-amino-propyl)-2-hydroxy-cyclohexylsulfanyl]-acétyl}-mutiline et le diastéréomère (1S,2S) de celle-ci
la 14-O-{[(6R,8R)-8-amino-1,4-dioxa-spiro[4.5]déc-6-ylsulfanyl]-acétyl}-mutiline et le diastéréomère (6S,8S) de celle-ci
la 14-O-{[4-amino-2-méthoxy-cyclohexylsulfanyl]-acétyl}-mutiline
et
la 14-O-{[5-amino-2-méthoxy-cyclohexylsulfanyl]-acétyl}-mutiline.

4. Composé destiné à être utilisé selon l'une quelconque des revendications 1 à 3, **caractérisé en ce que** le composé est la léfamuline.

5. Composé destiné à être utilisé selon l'une quelconque des revendications 1 à 4, **caractérisé en ce que** le composé se présente sous forme de sel et/ou de solvate.

6. Composé destiné à être utilisé selon l'une quelconque des revendications 1 à 5, **caractérisé en ce que** le composé est la léfamuline sous forme de sel d'acétate de léfamuline.

7. Composé destiné à être utilisé selon l'une quelconque des revendications précédentes, **caractérisé en ce que** la infection virale est une maladie respiratoire.

8. Composé destiné à être utilisé selon l'une quelconque des revendications précédentes, **caractérisé en ce que** la infection virale est un syndrome respiratoire aigu, tel que la grippe, le syndrome respiratoire aigu sévère (SRAS), le syndrome respiratoire du Moyen-Orient (SRMO) ou la COVID-19.

9. Composé destiné à être utilisé selon l'une quelconque des revendications précédentes, **caractérisé en ce que** le virus médiant l'infection virale est un virus à ARN monobrin à polarité positive ou négative, le virus étant choisi de préférence dans le groupe constitué par
- les Coronaviridae, y compris notamment le coronavirus humain,
- les Paramyxoviridae, y compris notamment les Paramyxovirinae tels que le virus de la rougeole et les Pneumovirinae tels que le virus respiratoire syncytial,
- les Orthomyxoviridae, y compris notamment le virus de la grippe,
- les Flaviviridae, y compris notamment le virus de la dengue et le virus Zika, et
- les Picornaviridae, y compris notamment le rhinovirus.

10. Composé destiné à être utilisé selon l'une quelconque des revendications précédentes, **caractérisé en ce que** la infection virale est une maladie transmise par l'air.
